Europäisches Patentamt

European Patent Office

Office européen des brevets

(19)

(11) Veröffentlichungsnummer : **0 419 885 B1**

# (12) EUROPÄISCHE PATENTSCHRIFT

(45) Veröffentlichungstag der Patentschrift :
**19.05.93 Patentblatt 93/20**

(51) Int. Cl.⁵ : **A23L 1/0526,** A23L 1/0532,
A23L 2/26

(21) Anmeldenummer : **90116778.3**

(22) Anmeldetag : **31.08.90**

(54) **Flüssige, stabile Guarkernmehl enthaltende Trink- und Sondennahrung.**

(30) Priorität : **31.08.89 DE 3928922**

(43) Veröffentlichungstag der Anmeldung :
**03.04.91 Patentblatt 91/14**

(45) Bekanntmachung des Hinweises auf die
Patenterteilung :
**19.05.93 Patentblatt 93/20**

(84) Benannte Vertragsstaaten :
**AT BE CH DE DK ES FR GB GR IT LI LU NL SE**

(56) Entgegenhaltungen :
**WO-A-86/05363**
**GB-A- 1 124 335**
**GB-A- 2 147 188**
**US-A- 4 390 550**

(56) Entgegenhaltungen :
**DERWENT WPIL Nr. 49, Zusammenfassung**
**Nr. 86-323831, 1986, Derwent Publications Ltd.,**
**London, GB; & JP - A - 61242541 (NIKKEN**
**FOOD HONSHA)**
**DERWENT WPIL Nr. 32, Zusammenfassung**
**Nr. 84-198108, Derwent Publications Ltd., Lon-**
**don, GB; & JP - A - 59112922 (A. ENDO)**

(73) Patentinhaber : **Resmer, Paul**
**Köbingerbergstrasse 22**
**W-8090 Wasserburg (DE)**

(72) Erfinder : **Resmer, Paul**
**Köbingerbergstrasse 22**
**W-8090 Wasserburg (DE)**

(74) Vertreter : **Brandes, Jürgen, Dr. rer. nat. et al**
**Wuesthoff & Wuesthoff, Patent- und**
**Rechtsanwälte, Schweigerstrasse 2**
**W-8000 München 90 (DE)**

**Beschreibung**

Bei der ernährungsmäßigen Behandlung von Störungen des Kohlenhydratstoffwechsels, insbesondere des Diabetes mellitus, gewinnt die Verwendung des Guarkernmehls aus den Früchten der indischen Büschelbohne steigende Bedeutung. Aufgrund des Gehalts an dem Hydrokolloid Galaktomannan bildet sich bei Einnahme einer gequollenes Guarkernmehl enthaltenden Zubereitung auf der Dünndarmschleimhaut eine hoch viskose dünne Filmschicht, wodurch die Resorptionsgeschwindigkeit von Nährstoffen verringert wird. Daher ist es möglich, durch gezielte Gaben an vorgequollenem Guarkernmehl vor einer Mahlzeit den Blutzuckerspiegel in den Spitzenwerten bei diabetisch veranlagten Patienten zu kontrollieren und egalisieren; vgl. Lembcke, B. et al, "Hepato-gastroenterol", Bd. 31, S. 183 - 186 (1984).

Neben diesem Effekt, der überwiegend durch seinen physikalischen Charakter erklärbar ist, sind noch eine Reihe weiterer ernährungsphysiologisch bedeutsamer Auswirkungen nach der Gabe von Guarkernmehl bekannt, so u.a. Senkung des Cholesterinspiegels im Blutserum sowie Inaktivierung der Pankreasamylase und damit vermindertem enzymatischen Angriff auf die Kohlenhydrate der Nahrung bei der Darmpassage. Als Verdickungsmittel im Speisebrei wirkt es viskositätserhöhend und damit appetithemmend. Auch reguliert es die Darmperestaltik und hat somit eine ausgezeichnete Ballaststoffunktion. Darüber hinaus können gewisse Fettstoffwechselstörungen mit Guarkernmehl bei einer Dosierung von ca. 10 - 15 g täglich erfolgreich behandelt werden.

Versuche, die bisher üblichen Einzelgaben von Guarkernmehl und das damit verbundene umständliche Vorquellen vor jeder Mahlzeit zu umgehen, indem man eine komplette fertige homogenisierte und sterilisierte Sonden- und Trinknahrung zur Verfügung stellt, die neben den notwendigen Nahrungsmittelbestandteilen das Hydrokolloid Guarkernmehl bereits enthält, hatten bisher keinen Erfolg. Zwar konnte durch Zugabe des Guarkernmehls zu einem flüssigen Nährstoffgemisch die Resorptionsgeschwindigkeit des hierbei als Kohlenhydratquelle eingesetzten Maltodextrins derart verlangsamt werden, daß der Glucosespiegel deutlich im Vergleich zur Standardmahlzeit absank, doch zeigte sich hierbei, daß sich in dieser vorgefertigten Zubereitung das eingebrachte Guarkernmehl bei der Lagerung nach relativ kurzer Zeit, d.h. nach ca. 8 - 14 Tagen, zu größeren Agglomeraten mit ca. 3 - 5 mm Durchmesser in den Sondennährlösungen zusammenlagerte und sogar teilweise auf der Oberfläche der Nährlösung als eine Art Kuchen aufschwamm. Auch bei derartigen Trinklösungen bildeten sich bei der Lagerung gallertartige, sagoähnliche Partikel, die dann bei der Einnahme der Zubereitung unangenehme Mundgefühle hervorgerufen haben. Das Zusammenlagern des Hydrokolloids ging hier teilweise so weit, daß sich größere zusammenhängende Fladen in der Nährlösung befanden, die sich dann auch durch intensives Schütteln der Flasche nicht zerteilen ließen.

Bei Versuchen, durch Zugabe eines anderen zusätzlichen Hydrokolloids zu der Guarkernmehl-haltigen Nährlösung eine Stabilisierung zu erreichen, damit die beschriebenen Effekte des Zusammenlagerns nicht mehr auftreten, zeigte sich, daß die meisten bekannten Hydrokolloide auch bei Verwendung höherer Konzentrationen nicht zu einer Besserung führten. So führte zwar der Zusatz von amidiertem Pektin, Apfelpektin, Carboxy-Methyl-Cellulose, Furcellaran, Johannisbrotkernmehl, Lambda-Carrageen, Methyl-Cellulose, Reismehl, Traganth und Xanthan zu einem synergistisch erhöhten Viskosewert der Zubereitung, bewirkte jedoch keine Stabilisierung des Guarkernmehls. Überraschenderweise konnte jedoch durch einen Zusatz von Natrium-Alginat und/oder Agar-Agar eine Stabilisierung erreicht werden.

Erfindungsgemäß enthält daher die Guarkernmehl-haltige Trink- und Sondennahrung zusätzlich Natrium-Alginat in einem Mengenbereich von 0,1 bis 5 g und/oder 0,25 bis 2 g Agar-Agar je Liter Lösung. Durch Verwendung des Natrium-Alginats wird die Abscheidung des Guarkernmehl-Hydrogels verhindert, so daß sich nicht mehr die oben erwähnten reiskorn- oder sagoähnlichen Partikelchen abscheiden und bei der Lagerung auf den Nahrungslösungen aufschwimmen. Das Guarkernmehlhydrokolloid bleibt gleichmäßig und praktisch nich mehr sichtbar in der Lösung verteilt, die daher auch für längere Zeit gelagert werden kann.

Bei den verschiedenen Natrium-Alginat-Typen hat das Verhältnis Guluronsäure zu Mannuronsäure in den Seitenketten des Alginats nicht nur einen Einfluß auf die Viskositätseigenschaften, sondern auch auf die stabilisierende Wirkung bei der Guarkernmehl-haltigen Lösung. Es dürften hier elektrostatisch abstoßende Kräfte der vorhandenen Carboxylgruppen des Alginathydrokolloids eine Rolle spielen. Vorzugsweise eignen sich hier Alginate, deren Guluronsäureanteil gegenüber dem Mannuronsäureanteil annähernd gleich ist im Vergleich zu herkömmlichen Alginattypen, bei denen der Mannuronsäureanteil überwiegt.

Eine derartige Natrium-Alginat-Type ist im Handel z.B. unter der Bezeichnung SATIALGINE SG 500 von der Fa. Sanofi Bio Industries GmbH, Düsseldorf, erhältlich.

Als besonders vorteilhaft hat sich ferner die Verwendung von Agar-Agar-Typen erwiesen, die aus Algen, z.B. Cracilaria-Rotalgen, gewonnen werden, die auf Feldern gezüchtet werden, im Unterschied zu solchen Agar-Agar-Typen, die aus an der Küste angeschwemmten Algen gewonnen werden. Aus auf Feldern gezüchteten Algen gewonnene Agar-Agar-Typen sind im Handel z.B. unter der Bezeichnung Aldagar TG 1 (Fa. O.Aldag, Hamburg) erhältlich.

Die erfindungsgemäßen Guarkernmehl-haltigen Trink- und Sondennährlösungen lassen sich gut stabilisieren und zeigen auch bei Lagerung über einen längeren Zeitraum von mindestens 6 Monaten keine Abscheidung von einer Guarkernmehlphase. Durch das Natrium-Alginat werden auch diätetisch wertvolle Effekte des Guarkernmehls noch verstärkt.

Da die abstoßenden Kräfte einerseits durch die Carboxylgruppen des Natrium-Alginats durch Kationen wie z.B. Na+, Ca++, Mg++ usw. in der Nährlösung wieder teilweise aufgehoben werden können, andererseits ein ernährungsphysiologisch sinnvoll aufgebautes Diätetikum auch CA++-Ionen in einer bestimmten Menge, z.B. 500 - 600 mg/ 1000 kcal, und andere Kationen beinhalten muß, kann durch Vorversuche die jeweils optimale Menge des Natrium-Alginats leicht ermittelt werden, um die gegenseitig beeinflussenden Effekte wieder weitgehend auszuschalten.

Im allgemeinen liegt ein solches Optimum für das Natrium-Alginat im Bereich von ca. 1,0 bis 2,5 g/l Flüssignahrung bei einer Menge von 5 bis 10 g Guarkernmehl je Liter.

Als Beispiele für stabile, flüssige diätetische Nährlösungen mit Guarkernmehl und Natrium-Alginat bzw. Agar-Agar werden nachfolgend je zwei Beispiele angegeben:


## 1.    Diabetes-Diät - flüssig

| Bestandteile: | Beispiel 1 Trinklösung in g/l | Beispiel 2 Sondenlösung in g/l |
|---|---|---|
| 1. Milchweiße | 50,0 | 50,0 |
| 2. pflanzliches Fett (z.B. Sojaöl) | 36,0 | 36,0 |
| 3. partiell abgebaute Maisstärke | 110,0 | 110,0 |
| 4. Zuckeraustauschstoff Sorbit | 10,0 | 10,0 |
| 5. Guarkernmehl | 5,0 | 5,0 |
| 6. Natrium-Alginat oder | 1,2 | 2,0 |
| 7. Agar-Agar | 1,5 | 1,0 |
| 8. Mineralsalze | 7,1 | 7,1 |
| 9. Spurenelemente (nur Fe) | 0,028 | 0,083 |
| 10. Vitamine | 0,17 | 0,17 |
| 11. Süßstoffmischung (Natreen[R]) | 0,30 | ----- |
| 12. Emulgatoren | 2,0 | 2,0 |
| 13. Aromastoffe (nat./nat. (id.) | 4,0 | ----- |
| 14. Antioxidationsmittel | 0,2 | 0,2 |
| 15. Trinkwasser | 834,002 | 837,447 |
| Gesamt: | 1060,000 g | 1060,000 g |
|  | ========== | ========== |

## 2. Reduktionsdiät - flüssig

| Bestandteile | Beispiel 3 in g/l | Beispiel 4 in g/l |
|---|---|---|
| 1. Milcheiweiße | 27,4 | 46,5 |
| 2. Magermilch | 616,0 | ---- |
| 3. pflanzliches Fett (Sojaöl) | 9,6 | 9,6 |
| 4. Maltodextrin DE 11-14 | 39,6 | 72,0 |
| 5. Mineralsalze | 3,6 | 3,6 |
| 6. Guarkernmehl | 6,0 | 6,0 |
| 7. Natrium-Alginat oder | 1,0 | 1,0 |
| 8. Agar-Agar | 1,5 | 1,0 |
| 9. Emulgatoren | 1,0 | 1,0 |
| 10. Vitamin-Mix | 0,56 | 0,56 |
| 11. Süßstoffmischung (Natreen [R]) | 0,44 | 0,44 |
| 12. Antioxidationsmittel | 0,12 | 0,12 |
| 13. Eisen-(II)-sulfat | 0,05 | 0,05 |
| 14. Trinkwasser | 343,00 g | 895,00 g |
| Gesamt: | 1050,00 g | 1037,00 g |

Diese Rezepturen der Beispiele 1 und 2 mit 4187 kJ = 1000 kcal entsprechen einer Nährstoffzusammensetzung von Eiweiß : Fett : Kohlenhydrate = 20 : 32 : 48 Energie-%. Ihr Stabilitätsverhalten ist gleich gut zu bewerten.

Die Rezeptur des Beispiels 3 stellt eine laktosehaltige und die Rezeptur des Beispiels 4 eine extrem laktosearme, flüssige Reduktionsdiät dar. Sie weisen pro 1000 ml 2345 kJ = 560 kcal auf. Sie entsprechen einer Nährstoffzusammensetzung von Eiweiß : Fett : Kohlenhydraten = 29 : 19 : 52 Energie - %.

Beide Reduktionsdiäten in flüssiger Form zeigen ebenfalls, wie die Nährlösungen im Falle 1 "Diabetes-Diät" eine ausgezeichnete Langzeitstabilität und gleichmäßige Verteilung des Guars über einen Zeitraum von mindestens 6 Monaten; erfahrungsgemäß jedoch von 10 bis 12 Monaten.

Die Herstellung der Rezepturen der Beispiele 1 bis 4 kann in einfacher Weise mittels nachfolgender Verfahrensstufen durchgeführt werden:

1. Vorquellphase des Guarkernmehles

Das für den Ansatz notwendige Guarkernmehl wird mit einem Teil des zum Ansatz kommenden Milcheiweißes vermengt und in einem Teil des notwendigen Ansatzwassers (bei 85°C) langsam eingerührt und so lange vorgequollen, bis eine dicke, weißliche Gallerte mit gleichbleibend hoher Viskosität entstanden ist.

2. Grundansatz der Hauptnährstoffe

In einem anderen Ansatzbehälter werden mit einem weiteren Teil des notwendigen Ansatzwassers (bei ca. 60°C) die Salze, das Alginat oder Agar-Agar, die Kohlenhydrate sowie die restlichen Eiweiße und der Emulgator langsam in den Behälter eingebracht und homogen und klumpenfrei verrührt.

3. Zusammenführen des Grundansatzes mit der Vorquellphase

Nach Fertigstellen der beiden vorgenannten Phasen werden diese mittels einer geeigneten Förderpumpe in einem Kessel unter ständigem Umpumpen zusammengeführt. Die Temperatur des Ansatzes liegt bei 70°C.

4. Herstellen des unsterilen Komplettansatzes

Es werden die noch fehlenden Rohstoffe wie Öl, Vitamine, Aromen, Süßstoffe und eventuell noch Spurenelemente in den Ansatzbehälter mittels geeigneter Emulgiergeräte eingebracht. Das unbeabsichtigte Einschlagen von Luft, welche sich schädlich auf vorgenannte Inhaltsstoffe auswirkt, sollte stets vermieden werden.

5. Vorsterilisation und Homogenisierung

Das noch unsterile Produkt wird umgehend, um eine ausreichende Sterilität zu erhalten einer Vorsterilisation mittels einem der bekannten UHT-Verfahren bei 135+/- 1°C und einer Erhitzungszeit von 8 - 10 Sek. unterworfen. Eine anschließende Hochdruckhomogenisierung bringt die notwendige Feinverteilung der Fetttröpfchen (ca. 0,5 μm bis 3 μm) und damit einen stabilen Emulsionsaufbau mit sich.

6. Abfüllung und Schlußsterilisation

Nach erfolgter Inprocesskontrolle und Ermittlung der wichtigsten Lebensmittel- und anderer Kennzahlen (wie z.B.: Trockenmasse, Fett, Eiweiß, Dichte, pH-Wert und Viskosität) wird das vorsterilisierte Produkt in handelsübliche Sterilmilchflaschen (500 ml/200 ml) abgefüllt, verschlossen und einer Schlußsterilisation unterworfen, derart, daß eine handelsübliche und während der Mindesthaltbarkeit ausreichende Sterilität erreicht wird. Nach abgeschlossener Qualitätskontrolle, die sich auf lebensmittelchemische, mikrobiologische, technische und physikalische Prüfmerkmale erstreckt und der erfolgten Freigabe, können die Produkte dem Handel zugeführt werden.

Das Produkt für die Sondennahrung hat eine Viskosität von ca. 70 cp (25°C) und besitzt für den Verwendungszweck eine ausreichende Fließfähigkeit (Sondengängigkeit) von ca. 300 ml/h bei einer hydrostatischen Druckhöhendifferenz von ca. 1 m. Der Einsatz und die Anwendung dieser standardisierten, Guarkernmehlhaltigen, bilanzierten Formuladiät kann unter Zuhilfenahme einer geeigneten Ernährungspumpe gesteigert oder anderweitig angepaßt werden.

Bei den aromatisierten Trinkvarianten spielt die Produktviskosität eine untergeordnete Rolle, doch haben die Lösungen nach Beispiel 1, 3 und 4 eine für die orale Anwendung sehr geeignete Viskosität, die einem guten sensorischen Gesamteinduck förderlich ist.

Alle Zubereitungen waren noch nach einer Lagerung von 6 Monaten unverändert stabil.

**Patentansprüche**

1. Flüssige, stabile Guarkernmehl enthaltende Trink- und Sondennahrung,
   **gekennzeichnet** durch einen zusätzlichen Gehalt an Natrium-Alginat in einer Menge von 0,1 bis 5 g je Liter und/oder Agar-Agar in einer Menge von 0,25 bis 2 g je Liter.

2. Trink- und Sondennahrung nach Anspruch 1,
   **gekennzeichnet** durch einen Gehalt von 1,0 - 2,5 Natrium-Alginat je Liter.

3. Trink- und Sondennahrung nach Anspruch 1 oder 2,
   **gekennzeichnet** durch einen Gehalt an gequollenem Guarkernmehl in einer Menge von 5 bis 10 g je Liter.

4. Trink- und Sondennahrung nach Anspruch 1 - 3,
   **gekennzeichnet** durch ein Natrium-Alginat, dessen Anteil an Guluronsäureresten im Molekül annähernd gleich groß ist wie der der Mannuronsäurereste.

5. Trink- und Sondennahrung nach Anspruch 1 bis 4, **gekennzeichnet** durch ein Agar-Agar, dessen funktionelle Eigenschaften und stabilisierende Wirkung auf das Guarkernmehl durch eine Züchtung von Agar-Agar liefernden Rotalgen auf sogenannten Algenfeldern zurückzuführen ist.

**Claims**

1. Liquid stable guar flour-containing beverage and tube nutrient,

characterized by an additional content of sodium alginate in an amount of 0.1 to 5 g per litre and/or agar-agar in an amount of 0.25 to 2 g per litre.

2.  Beverage and tube nutrient according to claim 1,
    characterized by a content of 1.0 - 2.5 sodium alginate per litre.

3.  Beverage and tube nutrient according to claim 1 or 2,
    characterized by a content of swollen guar flour in an amount of 5 to 10 g per litre.

4.  Beverage and tube nutrient according to claim 1 to 3,
    characterized by a sodium alginate of which the content of guluronic acid radicals in the molecule is approximately as great as that of the mannuronic acid radicals.

5.  Beverage and tube nutrient according to claims 1 to 4,
    characterized by an agar-agar having functional properties and a stabilizing effect on the guar flour which are due to cultivation of agar-agar-furnishing red algae on so-called algae fields.

**Revendications**

1.  Boisson et alimentation par sonde liquide contenant de la farine de germe de guar, caractérisé en ce qu'elle contient en outre de l'alginate de sodium en une quantité de 0,1 à 5 g par litre et/ou de l'agar-agar en une quantité de 0,25 à 2 g par litre.

2.  Boisson et alimentation par sonde de la revendication 1, caractérisée en ce qu'elle contient de 1,0 à 2,5 g d'alginate de sodium par litre.

3.  Boisson et alimentation par sonde selon la revendication 1 ou 2, caractérisée en ce qu'elle contient de la farine de germe de guar gonflée en une quantité de 5 à 10 g par litre.

4.  Boisson et alimentation par sonde selon les revendications 1-3, caractérisée en ce qu'elle contient de l'alginate de sodium dans la proportion par rapport aux esters de l'acide guluronique dans la molécule et est pratiquement égale à celle des esters de l'acide mannuronique.

5.  Boisson et alimentation par sonde selon les revendications 1 à 4, caractérisée par de l'agar-agar dont les propriétés fonctionnelles et l'action stabilisante sur la farine de germe de guar sont à attribuer à une culture d'algue rouge donnant de l'agar-agar dans ce qu'on appelle des champs d'algues.